(19) Europäisches Patentamt
European Patent Office
Office européen des brevets

(11) Numéro de publication: **0 001 193**
**B1**

(12) **FASCICULE DE BREVET EUROPEEN**

(45) Date de publication du fascicule de brevet: **14.10.81**

(51) Int. Cl.³: **C 07 C 149/26,**
C 07 C 101/36,
A 61 K 31/195

(21) Numéro de dépôt: **78400085.3**

(22) Date de dépôt: **31.08.78**

(54) **Amino-acides cycliques, leurs procédés d'obtention et compositions pharmaceutiques les contenant.**

(30) Priorité: **01.09.77 GB 3652577**

(43) Date de publication de la demande:
**21.03.79 Bulletin 79/6**

(45) Mention de la délivrance du brevet:
**14.10.81 Bulletin 81/41**

(84) Etats Contractants Désignés:
**BE CH DE FR GB NL SE**

(56) Documents cités:
**DE - A - 1 768 964**
**DE - B - 1 188 587**

**Chemical Abstracts vol. 80, n° 21, 27 May 1974, Columbus, Ohio, USA**
**A. W. COULTER et al "Structural and conformational analogs of L-methicnine as inhibitors of the enzymic synthesis of S-adenosyl-L-mothionine.III Carbocyclic and heterocyclic amino acids" page 170, colonne 2, abstract n° 117 621 w**

(73) Titulaire: **SCIENCE UNION ET Cie SOCIETE FRANCAISE DE RECHERCHE MEDICALE**
**14 rue du Val d'Or**
**F-92150 Suresnes (FR)**

(72) Inventeur: **Vincent, Michel**
**8, allée du Prunier Hardy**
**F-92220 Bagneux (FR)**
Inventeur: **Remond, Georges**
**9 Avenue des Etats Unis**
**78800 Versailles (FR)**
Inventeur: **Bure Jacques**
**22 rue Perronet**
**92200 Neuilly sur Seine (FR)**

(74) Mandataire: **Varady, Peter et al,**
**Service Brevets 22 rue Garnier**
**F-92200 Neuilly sur Seine (FR)**

**EP 0 001 193 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Amino-acides cycliques, leurs procédés d'obtention et compositions pharmaceutiques les contenant

La présente invention a pour objet de nouveaux aminoacides à chaine cyclique.

Elle a spécifiquement pour objet les (cycloalcoylène) amino acides et leurs dérivés répondant à la formule générale I

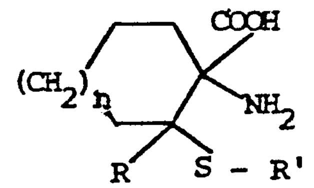

I

dans laquelle:

—R représente de l'hydrogène ou un radical alcoyle inférieur,

—R' représente de l'hydrogène, un radical alcoyle inférieur éventuellement substitué, un radical aryle monocyclique, un radical benzyle, un radical phényle substitué par un ou plusieurs substituants choisis dans le groupe constitué par un halogène, un alcoxy inférieur, un radical trifluorométhyle, un groupement amino, un groupement alcoylamino ou un groupement acylamino, ou un radical hétérocyclique,

—et n = 0 ou 1.

Parmi les composés de l'invention, on citera plus particulièrement à titre de composés actuellement préférés:

a) Les composés de formule générale $I_A$:

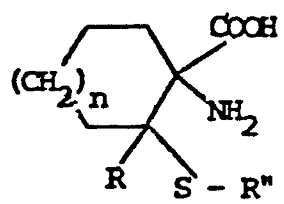

$I_A$

dans laquelle R et n sont définis comme ci-dessus, et R'' est un hydrogène, un radical alcoyle inférieur, un radical hydroxyalcoyle inférieur

b) Les composés de formule générale $I_B$

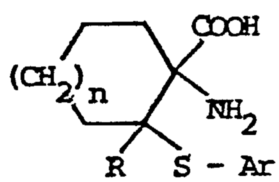

$I_B$

dans laquelle R et n sont définis comme précédemment, Ar est un radical aryle monocyclique comme un phényle non substitué ou substitué, un radical thiényle, un radical furyle, un radical thiazolyle et n représente zéro ou 1.

c) Les composés de formule générale $I_C$

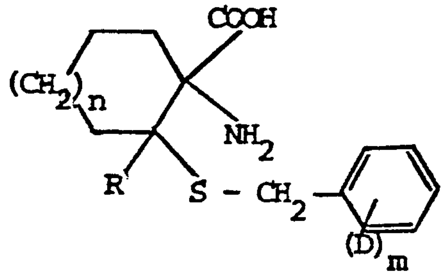

$I_C$

dans laquelle R et n sont définis comme précédemment, D est un halogène, un alcoxy inférieur, un radical trifluorométhyle, un groupement amino, un groupement alcoylamino ou un groupement acylamino, et m est égal à 0, 1.

Parmi les composés de formule générale I on pourra obtenir selon la signification de paramètre n un dérivé du cyclopentane (n = 0) ou du cyclohexane (n = 1).

On pourra citer notamment:

—l'acide (dl) 1-amino 2-mercapto cyclopentyl 1-carboxylique
—l'acide (dl) 1-amino 2-(thienylthio) cyclohexylcarboxylique
—l'acide (dl) 2-méthyl 2-benzylthio 1-amino cyclopentyl 1-carboxylique.
—l'acide (dl) 1-amino 2-(p.chlorophénylthio) cyclohexyl 1-carboxylique.
—l'acide (dl) 1-amino 2-benzylthio cyclopentyl 1-carboxylique.
—l'acide (dl) 1-amino 2-benzylthio cyclohexyl -1 carboxylique
—l'acide (dl) 1-amino 2-thio cyclohexyl 1-carboxylique.
—l'acide (dl) 1-amino 2-méthyl 2-benzylthio cyclohexyl 1-carboxylique.

L'invention s'étend aussi aux isomères optiques thréo ou érythro qui résultent de la présence de deux atomes de carbone asymétriques.

Les isomères thréo et érythro peuvent en outre être dédoublés en leurs antipodes optiques dextrogyre et levogyre. Ces isomères optiques sont également inclus dans l'invention.

L'invention a également pour objet les sels des composés de formule générale I avec un acide ou une base minérale ou organique, de préférence thérapeutiquement compatible.

Les composés de formule I sont amphotères et donnent des sels avec un acide comme bases minérale ou organique.

Parmi les sels avec un acide minéral ou organique, on citera plus particulièrement ceux thérapeutiquement compatibles comme par exemple les chlorohydrates, les borhydrates, les sulfates, les phosphates, les nitrates, les formiates, les acétates, les diphénylacétates, les dipropylacétates, les glycolates, les benzoates, les 3,5-dichlorobenzoates, les 3,4,5-triméthoxy benzoates, les nicotinates, les thiazoles carboxylates, les méthane sulfonates, les isothionates, les benzène sulfonates ou les glucose 1-phosphates.

Parmi les sels de base minérale, on pourra citer plus particulièrement les sels de métaux alcalins comme par exemple ceux de sodium, potassium, ammonium ou lithium; ceux de métaux alcalino-terreux comme ceux de calcium; ceux de magnésium, les sels neutres ou basiques d'aluminium, les sels de titane ou les sels ferreux.

Parmi les sels de base organique, on pourra citer notamment les sels d'amines aliphatique comme la diméthylamine, la triéthylamine, l'aminoéthanol ou le tri (hydroxyméthyl) aminoéthane; les sels d'amines arylaliphatiques comme la benzylamine, la dibenzylméthylamine, le $\beta$-méthyl phényléthylamine; les sels de bétaïnes ou d'ammoniums quaternaires comme la bétaïne ou la choline; des sels d'amino acides naturels ou artificiels comme la lysine, la valine, l'ornithine, la citrulline, la glutamine ou la serine; les sels avec les bases guanidiniques comme l'arginine, la glycocyamine, l'agmatine ou la créatinine; les sels avec les polypeptides comme les protamines, la salmine, la clupéine ou la caséine; les sels avec les aminosucres comme la glucosamine, la N-méthylglucamine ou la mannosamine.

Les sels formés avec des acides ou des bases non thérapeutiques peuvent servir de moyen d'isolement, de purification ou d'identification comme le perchlorate, le périodate ou le dinitrotoluate.

Les composés selon l'invention possèdent des propriétés pharmacologiques intéressantes notamment des propriétés immuno-dépressives, ils diminuent la résistance mécanique du tissu conjonctif néoformé dans le derme.

C'est ainsi que sur la souris CBA, on constate une diminution sensible de l'hypersensibilité de contact à l'oxazolone in vivo.

De ce fait les composés de formule générale I trouvent un emploi en thérapeutique humaine et vétérinaire soit comme agent immuno-dépres seur dans le traitement des maladies rhumatoïdes et des maladies a hypersécrétion protéique.

Les composés de formule générale I trouvent aussi un emploi dans le traitement des maladies dues à une croissance cellulaire rapide ou exagérée ou hypersécrétante. Ils peuvent encore trouver un emploi comme agent antibactérien seuls ou en association avec une sulfamide.

Enfin, ils peuvent être utilisés dans les cas de greffes d'organes, partiellement isogéniques.

A cette fin, les composés de formule générale I sont utilisés sous forme de compositions pharmaceutiques, en association avec un ou des excipients inertes non toxiques, pharmaceutiquement acceptables.

Les compositions pharmaceutiques peuvent contenir en outre des diluents, des supports absorbants, des agents liants, des agents protecteurs vis à vis des enzymes digestifs, des agents tampons ou des agents qui favorisent ou qui retardent la libération du principe actif.

On pourra citer à titre d'exemple le talc, les amidons, la zeine, le stéréate de magnésium, le phosphate de magnesie, la méthyl cellulose ou l'hydroxypropylcellulose comme excipients appropriés pour l'administration des formes solides, l'eau, les solutions salines ou le propylèneglycol pour les formes injectables, le beurre de cacao ou les stéarates de polyéthylèneglycol pour l'administration sous forme de suppositoires.

L'invention concerne également un procédé de préparation des composés de formule I qui consiste en ce que l'on soumet une cycloalcanone de formule II

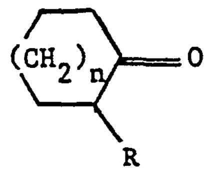

II

(dans laquelle R et n sont définis comme précédemment) à l'action d'un agent d'halogénation pour former le dérivé $\alpha$-halogéné de formule III

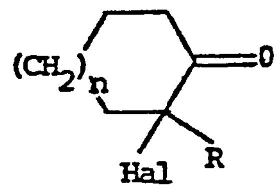

III

(dans laquelle R et n sont définis comme précédemment et Hal est un atome d'halogène autre que le fluor).

On fait réagir le composé III avec un thiol de formule R'SH pour former le dérivé de formule IV

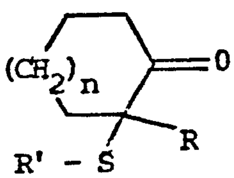

IV

(dans laquelle R, R' et n sont définis comme précédemment), puis on condense celui-ci avec un cyanure de métal alcalin et du carbonate d'ammonium dans les conditions de la réaction de Strecker pour former une spirohydantoïne de formule V

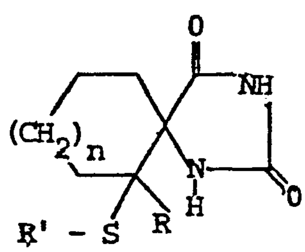

V

(dans laquelle la signification de R, R' et de n reste inchangée) on hydrolyse cette dernière en milieu alcalin ménagé et isole l'amino acide de formule I

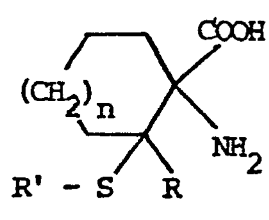

I

(dans laquelle R, R' et n sont définis comme précédemment) qu l'on traite par un métal alcalin dans l'ammoniac pour produire le thiol de formule VI

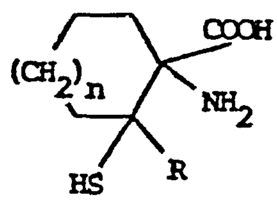

VI

(dans laquelle R et n gardent les significations antérieures).

Les composés de formule I comportent au moins deux atomes de carbone asymétrique et peuvent être dédoublés en leurs isomères optiques. Il est possible aussi de séparer les isomères thréo et érythro par des moyens physiques comme la chromatographie en phase liquide.

Les isomères érythro et thréo peuvent à leur tour être dédoublés en leurs isomères optiques par salification au moyen d'une base optiquement active comme la brucine, la strychnine, l'éphédrine, la pseudo éphédrine, la d-amphétamine ou la cinchonine, et cristallisation fractionnée du sel obtenu.

Le procédé selon l'invention peut en outre être défini par les modes d'exécution suivants:

1) l'hydrogénation de la cycloalcanone II est effectuée au moyen d'un agent d'halogénation comme un halogène tel que le chlore ou le brome; un N-halogéno alcoylamine, un N-halogéno arylamide tels que la N-chloro succinimide, la N-bromosuccininimide, la N-bromo acétamide, la N-bromobenzamide, la N-iodoacétamide, la N-bromophtalimide, la 5,5-dichlorodiphénylhydantoïne; un dérivé halogéné d'acide oxygéné tel que le chlorure de sulfuryle; un hypochlorite d'alcoyle comme l'hypochlorite de terbutyle; un perhalogénure de pyridinium tel que le perbromure de pyridinium.

2) la réaction d'halogénation de la cycloalcanone II est effectuée en solvant inerte, de préférence dans un solvant polaire comme le dioxane, la pyridine, la diméthylacétamide, l'hexaméthylphosphorotriamide, l'acide acétique, le sulfolane ou la diméthyl formamide.

3) la réaction de thiolation du dérivé III est effectuée en utilisant un dérivé alcalin du thiol.

4) l'hydrolyse de la spirohydantoine V est effectuée par un agent alcalin tel que l'hydroxyde de sodium ou l'hydroxyle de baryum.

5) la formation du thiol VII est obtenue à partir du composé VI par hydrogénolyse, notamment par un métal alcalin tel que le sodium, le potassium ou le lithium, dans l'ammoniac liquide.

L'invention s'étend aussi aux composés intermédiaires formés au cours de la synthèse des composés de formule I

1) les $\alpha$-halogénocétones de formule III

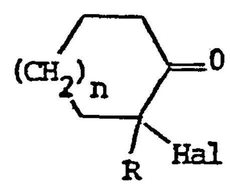

III

dans laquelle R et n sont définis comme précédemment et Hal représente un atome d'halogène autre que le fluor.

2) les cycloalcanones de formule IV

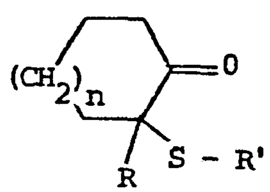

IV

dans laquelle R, R' et n sont définis comme précédemment

3) les spiro hydantoïnes de formule V

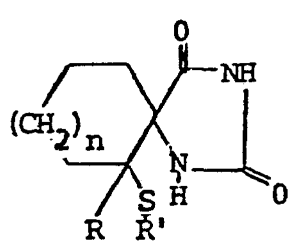

V

dans laquelle R, R' et n sont définis comme précédemment.

Les exemples suivants servent à illustrer l'invention. Ils ne la limitent en aucune façon.

Exemple I

Acide (dl) 1-amino 2-méthyl 2-benzylthio cyclohexyl carboxylique.

*Stade A*

2-méthyl 2-chloro cyclohexanone.

Après avoir dissout 112 g de 2-méthyl cyclohexanone dans 500 ml de tétrachlorure de carbone, on ajoute goutte à goutte une solution de 90 ml de chlorure de thionyle dans 150 ml de tétrachlorure de carbone. L'addition dure 60 min. et s'effectue sous forte agitation en maintenant le température du milieu réactionnel à 15°C en viron à l'aide d'un bain d'eau. L'agitation est ensuite maintenue pendant une période supplémentaire de deux heures. Le mélange réactionnel est alors lavé à trois reprises avec l'acide chlorhydrique dilué, puis à deux reprises avec une solution saturée de bicarbonate de sodium et enfin avec une solution saturée de chlorure de sodium. La phase organique est séparée, séchée sur sulfate de sodium, filtrée puis évaporée à sec sous pression réduite. On obtient un résidu sec pesant 180 g environ que l'on purifie par distillation fractionnée. La fraction principale distille à 85—90°C sous 2,66kPa (20mmH)

$n_D^{25}$= 1,4672

*Stade B*

2-méthyl 2-benzylthio cyclohexanone.

On dissout 126,5 g de 2-méthyl 2-chloro cyclohexanone obtenue au stade précédent dans 70 ml d'éthanol. On y ajoute un mélange de 82 g de benzylmercaptan et de 26,4 g de soude en solution dans 330 ml d'éthanol. Le mélange est porté au reflux pendant 15 min. On laisse ensuite revenir le mélange à la température ordinaire et on évapore l'éthanol. Le résidu huileux est repris dans 400 ml d'eau et on épuise la suspension avec 3 fois 25 ml de chlorure de méthylène. Les phases organiques sont réunies, lavées à l'eau jusqu'à neutralité puis séchées, filtrées et évaporées à sec. Le résidu est purifié par distillation fractionnée. On obtient ainsi 120 g de 2-méthyl 2-benzylthio cyclohexanone qui distille à 142—144°C (rendement 78%).

*Stade C*

2-méthyl 2-benzylthioxyclohexane spirohydantoïne.

Dans une fiole à bouchage hermétique on introduit successivement 2,5 g de cyanure de potassium préalablement dissout dans 15 ml d'eau puis un mélange de 11.7 g de 2-méthyl 2-benzylthio cyclohexanone, 0,6 g de carbonate d'ammonium et 40 ml d'éthanol à 60%. L'addition dure environ 15 min. Le mélange est placé dans un bain thermostatique à 50°C et maintenu à cette température pendant 45 heures sous agitation. Après ce temps, le solvant de la réaction est évaporé sous pression réduite et le résidu sec est mis en suspension dans un mélange de 180 ml d'eau et 100 ml d'éther. On agite vigoureusement et on isole le précipité par filtration. On le lave à l'eau et on le sèche à 50°C. On isole ainsi 2,5 g de spirohydantoïne fondant à 210°C. La concentration des eaux mères permet d'isoler une seconde fraction pesant 0,9 g. Le rendement global est d'environ 20%.

*Stade D*

Acide (dl) 1-amino 2-methyl 2-benzylthio cyclohexyl 1-carboxylique.

On prépare une solution de 63,1 g d'hydroxyde de baryum dans 500 ml d'eau à chaud. On filtre l'insoluble après retour à la température ordinaire. On ajoute le filtrat à une suspension de 30,5 g de la spirohydantoïne obtenue au stade C dans 150 ml d'eau. On complète ensuite le volume à 1000 ml. Le récipient est hermétiquement fermé et chauffé à 140°C pendant 65 h. On laisse ensuite revenir la température intérieure à 90°C et on transvase le liquide dans une fiole conique. Les parois de récipient hermétique sont rincées à deux reprises avec une solution N d'acide chlorhydrique. Les solutions acides sont ajoutées au mélange réactionnel. On acidifie alors l'ensemble des liqueurs à pH 1 par addition d'acide Chlorhydrique.

On neutralise ensuite par addition de bicarbonate de sodium jusqu'à un pH de 6,3. Il apparait un insoluble et le mélange est laissé une nuit en glacière. On épuise la suspension au chloroforme. On sépare les phases chloroformiques que l'on filtre et évapore à sec. Le résidu cristallin est repris dans de l'acide chlorhydrique N et chromatographié sur une colonne garnie de résine carboxylique échangeuse d'ions (DOWEX XW8)®. Après écoulement du solvant, on élue l'aminoacide par une solution aqueuse de triéthylamine jusqu'à disparition de la réaction colorée à la ninhydrine.

Après évaporation de éluats sous pression réduite, on obtient 7 g d'acide (dl) 2-benzylthio 2-méthyl 1-amino cyclhexyl 1-carboxylique (rendement 25%). Le produit est recristallisé de l'acétone. Son point de fusion est de 215°C.

Analyse: $C_{15}H_{21}NO_2S$ = 279,40

| | C | H | N | S % |
|---|---|---|---|---|
| Calculé | 64,48 | 7,57 | 5,01 | 11,47 |
| Trouvé | 64,20 | 7,20 | 5,00 | 11,32 |

Ce composé retient l'eau très énergiquement et la deshydratation nécessite un chauffage à 150°C pendant une heure.

Exemple II

Acide (dl) 1-amino 2-benzylthio cyclohexyl 1-carboxylique

En utilisant le mode opératoire de l'exemple I au départ de la cyclohexanone, on obtient successivement:

—1a 2-benzylthiocyclohexanone $Eb_{0,07}$ = 128—132°C (Rendement 72%).

—1a 2-benzylthiocyclohexane spirohydantoïne F = 126°C

—l'acide (dl) 1-amino 2-benzylthio cyclohexyl -1 carboxylique (mélange d'isomères) F= 220°C.

Analyse: $C_{14}$ $H_{19}$ $NO_2S$ = 265,377

| | C | H | N | S % |
|---|---|---|---|---|
| Calculé | 63,40 | 7,35 | 5,29 | 12,09 |
| Trouvé | 62,93 | 7,03 | 5,22 | 11,92 |

Ce composé est soluble dans l'acide chlorhydrique dilué. L'évaporation de la solution fournit le chlorhydrate.

Exemple III

Acide (dl) 1-amino 2-thio cyclohexyl 1-carboxylique.

A 100 ml d'ammoniac liquide à — 30°C et contenant 6 g d'acide 1-amino 2-benzylthiocyclohexyl 1-carboxylique obtenu à l'exemple II, on ajoute 3,2 g de Sodium fraichement coupé en lamelles, par petites portions jusqu'à ce que le mélange prenne une coloration bleue stable. Le mélange est maintenu sous agitation pendant une heure. L'excès de réactif est décomposé par addition ménagée de chlorure d'ammonium. On laisse ensuite l'amoniac s'évaporer à température ordinaire. Le résidu sec est repris par quelques ml d'eau et la suspension est amenée à pH 2 par addition d'acide chlorhydrique N.

On évapore ensuite à sec. Le résidu est lavé à l'acétone puis recristallisé de l'isopropanol. On obtient ainsi 3 g de chlorhydrate hemihydraté qui fond au dessus de 250°C.

Analyse: $C_7$ $H_{14}$ cl $NO_2S$, 1/2 H20 = 220,720

| | C | H | N | cl | S |
|---|---|---|---|---|---|
| Calculé | 38,09 | 6,85 | 6,34 | 16,06 | 14,52 |
| Trouvé | 34,14 | 6,37 | 6,34 | 16,42 | 14,35 |

Exemple IV

Acide (dl) 1-amino 2-(p.chlorophenylthio) cyclohexyl 1-carboxylique.

En utilisant le mode opératoire de l'exemple I au départ de la Cyclohexanone et en remplaçant le benzylmercaptan par le p.chlorophenylthio on obtient l'acide (dl) 1-amino 2-(p.chlorophenylthio) cyclohexyl 1-carboxylique (mélange d'isomères). Il fond au-dessus de 250°C (avec decomposition).

Analyse: $C_{13}$ $H_{16}$ Cl $NO_2S$ = 285,8

| | C | H | N | Cl | S % |
|---|---|---|---|---|---|
| Calculé | 54,64 | 5,64 | 4,90 | 12,41 | 11,22 |
| Trouvé | 54,26 | 5,49 | 4,89 | 12,50 | 11,40 |

Exemple V

Acide (dl) 1-amino 2-benzylthiocyclopentyl 1-carboxylique.

En opérant selon le mode opératoire de l'exemple I au départ de la cyclopentanone, on obtient l'acide (dl) 1-amino 2-benzylthiocyclopentyl 1-carboxylique sous forme de monohydrate.

Analyse de l'hydrate: $C_{13}$ $H_{17}$ $O_2NS.H_2O$ = 269

| | C | H | N | S % |
|---|---|---|---|---|
| Calculé | 57,97 | 7,10 | 5,20 | 11,90 |
| Trouvé | 58,69 | 7,05 | 5,20 | 12,46 |

Exemple VI

Acide (dl) 2-méthyl 2-benzylthio 1-amino cyclopentyl 1-carboxylique.

La 2-méthylcyclopentanone est convertie en acide (dl) 2-méthyl 2-benzylthio 1-amino cyclopentyl 1-carboxylique en opérant selon le mode opératoire de l'exemple I. Il fond au-dessus de 250°C (avec décomposition).

Analyse: $C_{14}$ $H_{19}$ $NO_2S$ = 265,38

| | C | H | N % |
|---|---|---|---|
| Calculé | 63,36 | 7,22 | 5,28 |
| Trouvé | 63,34 | 7,07 | 5,48 |

Exemple VII

Acide (dl) 1-amino 2-(thienylthio)cyclohexyl-1 carboxylique.

Et utilisant le mode opératoire de l'exemple I au départ de 2-chlorocyclohexanone et de 2-thiénylthiol, on obtient l'acide (dl) 1-amino 2-(thienylthio)cyclohexyl 1-carboxylique.

Ce composé fond au-dessus de 250°C.

Analyse: $C_{11}$ $H_{15}$ $NO_2S$ = 257,378

| | C | H | N | S % |
|---|---|---|---|---|
| Calculé | 51,33 | 5,87 | 5,44 | 24,91 |
| Trouvé | 51,42 | 6,04 | 5,70 | 24,81 |

Exemple VIII

Acide (dl) 1-amino 2-thiocyclopentyl 1-carboxylique.

A 900 ml d'ammoniac maintenus à 70°C, on ajoute 20 g d'acide (dl) 1-amino 2-benzylthio cyclopentyl 1-carboxylique en une fois, puis par petites fractions 8 g de sodium en fragments fraichement découpés. Le mélange prend une coloration bleu-foncé. On laisse reposer pendant une heure puis on décolore le mélange réactionnel par addition ménagée de chlorure d'ammonium. On laisse ensuite l'ammoniac s'évaporer à température ordinaire. Le résidue est disous dans l'eau et acidifié par de l'acide chlorhydrique jusqu'à pH 2. La solution acide est chromatographiée sur une colonne de résine échangeuse d'ions DOWEX 50W8® préalablement traitée à l'acide chlorhydrique et lavée à l'eau. Après passage de l'effluent, on élue l'acide 1-amino 2.thiocyclopentyl -1-carboxylique par une solution aqueuse de triéthylamine à 50 g/litre. On recueille les éluats en 16 fractions de 25 ml. Après évaporation de chaque fraction on recueille le thiol dans les fractions 9 à 16. Le thiol est lavé à l'éther puis à l'éthanol. On obtient ainsi 4,3 g d'acide (dl) 1-amino 2-thiocyclopentyl 1-carboxylique (Rendement 27%).

Analyse: $C_6$ $H_{11}$ $NO_2S$ = 161,32

| | C | H | N | S % |
|---|---|---|---|---|
| Calculé | 44,67 | 6,87 | 8,68 | 19,87 |
| Trouvé | 44,52 | 7,02 | 8,55 | 19,57 |

Exemple IX

Séparation des diastéréoisomères de l'acide 1-amino 2-benzylthiocyclopentyl 1-carboxylique.

On met 3,5 g d'acide 1-amino 2-benzylthio cyclopentyl 1-carboxylique préparés dans l'exemple V en solution dans un mélange de chloroforme et de méthanol.

On injecte la solution dans un chromatographe Haute pression en phase liquide équipé d'une colonne garnie de 1,5 kg de silice (10—40 $\mu$).

On sépare les deux diastéréoisomères par élution à l'aide d'un mélange chloroforme/éthanol/acide acétique. Ils sont de nouveau purifiés par une seconde chromatographie en phase liquide préparative (colonne de silice 5.20 $\mu$ RP8) et élution au méthanol. Finalement, on recristallise chaque diastéréoisomère d'un mélange chloroforme/méthanol/éther.

On recueille le premier isomère en deux fractions pesant au total 2 g (isomère $\alpha$). Le second

isomère est également obtenu en 2 fractions (poids total 0,9 g isomère $\beta$).

*Isomère $\alpha$*: point de fusion au-dessus de 250°C soluble dans les solutions aqueuse N/10 d'acide méthane sulfonique.

Analyse: $C_{13}H_{17}NO_2S = 251.35 + 2,5\%$ eau

| | C | H | N | S % |
|---|---|---|---|---|
| Calculé | 62,12 | 6,82 | 5,57 | 12,76 |
| Trouvé | 62,00 | 6,72 | 5,68 | 13,18 |

*Isomère $\beta$*: point de fusion au-dessus de 250°C

Analyse: $C_{13}H_{17}NO_2S = 251.35$

| | C | H | N | S % |
|---|---|---|---|---|
| Calculé | 62,12 | 6,82 | 5,57 | 12,76 |
| Trouvé | 62,01 | 7,17 | 5,60 | 12,73 |

## Exemple X

Séparation de deux diastéréoisomères de l'acide 1-amino 2-méthyl 2-benzylthio cyclohexyl 1-carboxylique.

Les deux diastéréoisomères $\alpha$ et $\beta$ sont séparés comme dans l'exemple IX à partir de 3 g d'acide 1-amino 2-méthyl 2-benzylthio cyclohexyl 1-carboxylique préparé dans l'exemple I stade D.

*Isomère $\alpha$*: point de fusion 262°C (avec décomposition).

Analyse: $C_{15}H_{21}NO_2S = 279.40$

| | C | H | N | S % |
|---|---|---|---|---|
| Calculé | 64,48 | 7,58 | 5,01 | 11,48 |
| Trouvé | 64,39 | 7,39 | 5,14 | 11,82 |

*Isomère $\beta$*: point de fusion: 260°C (avec décomposition)

Analyse: $C_{15}H_{21}NO_2S = 279.40$

| | C | H | N | S % |
|---|---|---|---|---|
| Calculé | 64,48 | 7,58 | 5,01 | 11,48 |
| Trouvé | 64,39 | 7,41 | 5,15 | 11,62 |

## Exemple XI

Séparation des diastéréoisomères de l'acide 1-amino 2-p chlorophénylthio cyclohexyl 1-carboxylique.

Au départ de 5 g d'acide (dl) 1-amino 2.p.chlorophényl thio cyclohexyl 1-carboxylique préparés dans l'exemple IV et en utilisant la technique de l'exemple IX, on sépare 3 g d'isomère. L'isomère $\beta$ n'a pas été obtenu à l'état de pureté analytique. L'isomère $\alpha$ est transformé en chlorhydrate par dissolution dans l'acide chlorhydrique normal et évaporation à sec.

Analyse: $C_{13}H_{17}Cl_2NO_2S = 322.27$

| | C | H | N | S | Cl |
|---|---|---|---|---|---|
| Calculé | 48,45 | 5,32 | 4,35 | 9,95 | 22,00 |
| Trouvé | 48,30 | 5,37 | 4,67 | 9,91 | 22,65 |

## Exemple XII

Acide (dl) 1-amino 2-méthylthio 2-méthyl cyclohexyl -1 carboxylique.

Au départ de la 2-méthylcyclohexanone et du méthylmercaptan, on obtient selon le mode opératoire de l'exemple I l'acide (dl) 1-amino 2-méthyl 2-méthylthiocyclohexyl 1-carboxylique.

Point de fusion: 225°C (décomposition)

Analyse: $C_9H_{17}NO_2S = 203.31$

| | C | H | N | S % |
|---|---|---|---|---|
| Calculé | 53,17 | 8,43 | 6,89 | 15,77 |
| Trouvé | 53,24 | 8,59 | 6,97 | 15,93 |

Exemple XIII

Au départ de la 2-méthylcyclohexanone et de l'hydroxyéthyl mercaptan on obtient selon le mode opératoire de l'exemple I l'acide (dl) 1-amino 2-(2-hydroxyéthylthio)2-méthyl cyclohexyl 1-Carboxylique.

Point de fusion: 246°C (décomposition)

Analyse: $C_{10}$ $H_{19}$ $NO_3S$ = 233.33

| | C | H | N | S |
|---|---|---|---|---|
| Calculé | 51,46 | 8,21 | 6,00 | 13,74 |
| Trouvé | 51,22 | 8,35 | 6,16 | 13,79 |

Exemple XIV

Comprimés renfermant 250 mg d'acide 2-méthyl 2-benzylthio 1-aminocyclohexyl 1-carboxylique (mélange de diastéréoisomères).

| | |
|---|---|
| Acide 2-méthyl 2-benzylthio 1-amino Cyclohexyl 1-carboxylique | 2,500 kg |
| amidon de maïs | 0,250 kg |
| amidon de blé | 0,250 kg |
| éthyl cellulose | 0,040 kg |
| silicate de magnésium | 0,150 kg |
| talc | 0,100 kg |
| caséine formolée | 0,025 kg |
| lactose | 0,200 kg |

pour 10.000 comprimés d'un poids moyen de 0,35 g

Exemple XV

Etude pharmacologique des composés selon l'invention.

Les produits selon l'invention sont des acide $\alpha$-aminés $\beta$-thio ou thioester qui ont été synthétisés et testés dans la perspective de produits actifs sur le tissue conjectif: sur la *polymérisation du collagène* par une mécanisme analogue à la D-pénicillamine selon les descriptions de JAFFE et NIMMI et sur *l'action immunodépressive* de la D-Pénicillamine, par un mécanisme différent de celui des corticoïdes, des agents alcoylants ou des antimétabolites.

Les composés selon l'invention ont été testés principalement dans ces deux directions. Dans cette série on a trouvé des produits d'activité immunodépressive: leur mode d'action se rapproche soit de la D-Pénicillamine soit des antimétabolites, d'autres produits actifs sur le tissu conjonctif et également immunodépresseurs.

TECHNIQUE

*Immunodépression:* le test d'hypersensibilité retardée de contact à l'Oxazolone décrit par Phanuphak et Coll (J. of Immunol 1974, 112—115) a été modifié de manière à obtenir une réponse cellulaire T pratiquement pure. En bref, la sensibilisation des souris (CD O 25—27 g) s'effectue par application percutanée dorsale avec une solution oléoacétonique (haile d'olive pur 1 vol. pour 4 vol. d'acétone) à $3\%_0$ d'oxazolone; le challenge a lieu 5 jours après, également par contact au niveau de l'oreille, avec une solution à 3% d'oxazolone dans le même solvant. La réaction est lue 24 et 48 h après. L'épaisseur de l'infiltration est mesurée, le nombre d'animaux répondeurs au-dessous d'un seuil, est noté. Les animaux sont traités soit 3 fois 2 jours et 1 jour avant ainsi que le jour de la sensibilisation

(j −2,1,0), soit à dose unique: 2 jours après la sensibilisation (j+2)).

*Synthèse et polymérisation du collagène:* elles sont étudiées globalement par la résistance physique à l'élongation des tissus, mesurée par jauge selon la technique de JAFFE et Coll (Science 1968, *161* 1016). La résistance du conjonctif néoformé post-cicatriciel est mesuré 8 jours après la lésion, le traitement ayant lieu du 5ème au 7ème jour; la résistance du derme natif est mesurée après 9 jours de traitement.

RESULTATS

*Activité immunodépressive:* pour les produits de référence l'activité est retrouvée à dose élevée, mais nous avons à faire à des traitements par dose unique (j+2) ou relativement courts (j−2, −1,0) alors que ces produits nécessitant une certaine imprégnation pour manifester leurs effets. Deux types d'activité sont retrouvés:

1) En cours de sensibilisation (j+2) pour les antimétabolistes, les agents alcoylants et les corticoïdes.

2) Avant la sensibilisation (j−2,−1,0) pour la D-Penicillamine et la cycloleucine.

Les composés selon l'invention aux doses de 50 à 200 mg/kg possèdent une action soit en cours de sensibilisation soit avant sensibilisation.

Activité sur le tissu conjonctif: (synthèse et polymérisation du collagène)

On peut établir l'activité de la D-Penicillamine sur ce test comme substance de référence à la dose de 250 mg/kg. Les cortisoniques possèdent également une efficacité. Par contre les autres métabolites et les agents alcoylants n'ont d'effet qu'à dose toxique.

Les composés selon l'invention et notamment l'acide 1-amino 2-benzylthio 2-méthylcyclohexyl 1-carboxylique sont plus actifs sur le collagène natif que sur la collagène néoformé. Ils agissent à partir de la dose de 250 mg/kg per os.

*Détermination de la toxicité aigüe:*

Le composés selon l'invention ont été administrés par voie buccale à doses croissantes à des lots de 10 souris (souche CD) pesant 20 g environ. Les animaux sont placés en observation pendant 15 jours et les morts, s'il y en a, sont dénombrés. La dose léthale 50 varie selon les produits entre 1 et plus de 2 g. A titre de comparaison la $DL_{50}$ de la D-Penicillamine est de 8 g environ per os.

**Revendications**

1. Les amino acides cycliques α-substitués de formule I

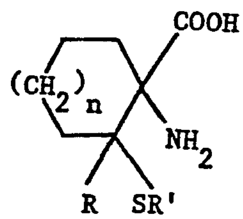

I

dans laquelle:

—R représente de l'hydrogène ou un radical alcoyle inférieur,

—R représente de l'hydrogène, un radical alcoyle inférieur éventuellement substitué par un hydroxyle, un radical aryle monocyclique, un radical benzyle, un radical phényle substitué par un ou plusieurs substituants choisis dans le groupe constitué par un halogène, un alcoxy inférieur, un radical trifluorométhyle, un groupement amino, un groupement alcoylamino ou un groupement acylamino, ou un radical hétérocyclique,

—et n = 0 ou 1.

2. Les sels des composés de formule I selon la revendication 1, avec un acide minéral ou organique, ou bien avec une base minérale ou organique de préférence thérapeutiquement compatibles.

3. Les diastéréoisomères érythro et threo des composés de formule I selon la revendication 1 ainsi que leurs formes optiquement actives.

4. L'acide 1-amino 2-benzylthio cyclopentyl 1-carboxylique.

5. L'acide 1-amino 2-mercapto cyclohexyl 1-carboxylique.

6. L'acide 2-méthyl 2-benzylthio 1-aminocyclopentyl 1-carboxylique.

7. L'acide 1-amino 2-benzylthio cyclohexyl 1-carboxylique.

8. L'acide 1-amino 2-(p.chlorophenylthio) cyclohexyl 1-carboxylique.

9. L'acide 1-amino 2-(thiénylthio) cyclohexyl -1-carboxylique.

10. L'acide 1-amino 2-mercapto cyclopentyl 1-carboxylique.

11. L'acide 1-amino 2-méthyl 2-benzylthio cyclohexyl 1-carboxylique.

12. L'acide 1-amino 2-méthylthio 2-méthylcyclohexyl 1-carboxylique.

13. L'acide 1-amino 2-(2-hydroxyéthylthio) 2-méthyl cyclohexyl 1-carboxylique.

14. Compositions pharmaceutiques comprenant un excipient ou un véhicule inerte non-toxique thérapeutiquement acceptable, caractérisées par une teneur en au moins un composé selon la revendication 1 ou un de ses sels d'addition avec un acide ou une base comme principe actif.

15. Compositions pharmaceutiques selon la revendication 14 caractérisées en ce que la teneur en principe actif est comprise entre 100 mg et 400 mg par prise unitaire.

16. Un procédé d'obtention des composés de formule I selon la revendication 1

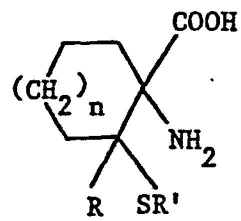

dans laquelle R, R' et n sont définis comme dans la revendication 1.
caractérise en ce que l'on soumet une cycloalcanone de formule II

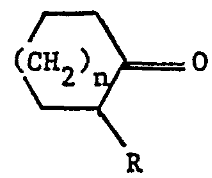

II

(dans laquelle R et n ont les significations antérieures) à l'action d'un agent d'halogénation pour former une $\alpha$-halogéno cétone de formule III

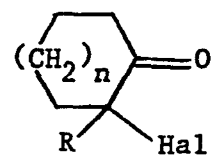

III

(dans laquelle Hal est un halogène autre que le fluor et R et n ont les significations antérieures, que l'on fait réagir avec un mercaptan de formule

R' — SH

(dans laquelle R' est un radical alcoyle inférieur, un radical aryle ou un radical benzyle) pour former le dérivé thio de formule IV

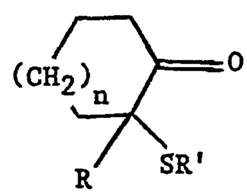

IV

(dans laquelle les substituants R, R' et n sont définis comme précédemment); on fait réagir ce dernier avec un cyanure de métal alcalin et un sel d'ammonium dans les conditions de la Réaction de Strecker pour former une spiro hydantoïne de formule V

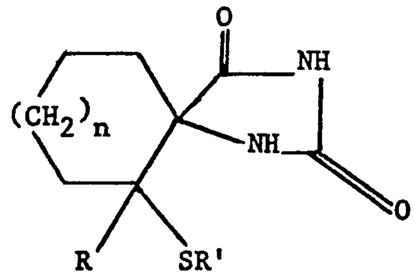

V

(dans laquelle R, R′ et n ont les significations antérieures) hydrolyse cette dernière en milieu alcalin pour fournir un amino acide de formule I

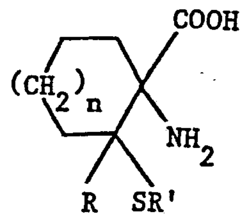

I

(dans laquelle les substituants R, R′ et n sont définis comme précédemment) que l'on soumet, le cas échéant, à une hydrogénolyse pour former un dérivé thio de formule VI

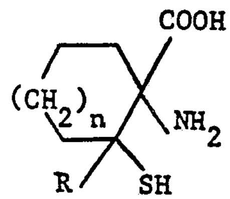

VI

17. Un procédé selon la revendication 16 dans lequel le composé amphotère de formule I est converti en un sel par addition d'un acide minéral ou organique ou d'une minérale ou organique.

18. Un procédé selon la revendication 16 dans lequel on sépare les isomères des composés de formule I par une méthode physique, telle que la cristallisation fractionnée ou la chromatographie.

**Claims**

1. α-substituted cyclic amino acids of formula I

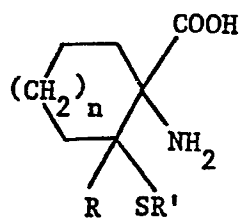

(I)

in which:

R denotes hydrogen or a lower alkyl radical,

R′ denotes hydrogen, a lower alkyl radical, a hydroxy-substituted lower alkyl radical, a monocyclic aryl radical, a benzyl radical, a substituted phenyl radical in which the substituent(s) is (are) selected from the group consisting of halogen, lower alcoxy, trifluoromethyl, amino, alkylamino or acylamino, or a heterocyclic radical,

n is 0 or 1.

2. The salts of the compounds of formula I according to claim 1 with a mineral or organic acid, or with a mineral or organic base, which are preferably therapeutically compatible.

3. The erythro and threo diastereoisomers of the compounds of formula I according to claim 1 and their optical isomers.

4. 1-amino-2-benzylthio-cyclopentyl-1-carboxylic acid.

5. 1-amino-2-mercapto-cyclohexyl-1-carboxylic acid.

6. 2-methyl-2-benzylthio-1-aminocyclopentyl-1-carboxylic acid.
7. 1-amino-2-benzylthio-cyclohexyl-1-carboxylic acid.
8. 1-amino-2-(p-chlorophenylthio)-cyclohexyl-1-carboxylic acid.
9. 1-amino-2-(thienylthio)-cyclohexyl-1-carboxylic acid.
10. 1-amino-2-mercapto-cyclopentyl-1-carboxylic acid.
11. 1-amino-2-methyl-2-benzylthio-cyclohexyl-1-carboxylic acid.
12. 1-amino-2-methylthio-2-methylcyclohexyl-1-carboxylic acid.
13. 1-amino-2-(2-hydroxyethylthio)-2-methyl-cyclohexyl 1-carboxylic acid.
14. Pharmaceutic compositions comprising an inert non-toxic therapeutically acceptable excipient or carrier, which contain at least one compound according to claim 1 or an addition salt with acid or a base as active ingredient.
15. Pharmaceutic compositions according to claim 14 wherein the unit dosage of the active ingredient is comprised of between 100 and 400 mg.
16. A process for preparing compounds of formula I according to claim 1

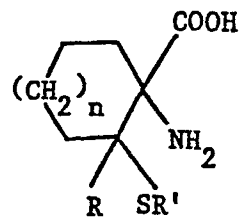

(I)

in which:

R, R′ and n have the same meaning as in claim 1 wherein a cycloalcanone of formula II

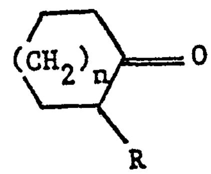

(II)

(in which:

R and n have the above meaning) is submitted to the action of a halogenating agent to produce a α-halogeno ketone of formula III:

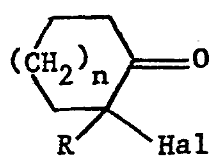

(III)

(in which:

Hal is a halogen with the exception of the fluorine and R and n have the above meaning) which is reacted with a thiol of formula R′ — SH (in which R′ is a lower alkyl radical, an aryl radical or a benzyl radical) so as to produce a thio derivative of formula IV:

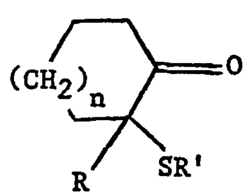

(IV)

in which:

the substituents R, R′ and n have the above-given definition;

the latter is reacted with an alkali metal cyanide and ammonium carbonate under the conditions of Strecker reaction so as to produce a spiro hydantoine of formula V:

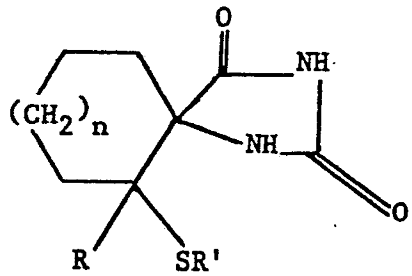

(V)

(in which R, R′ and n have the above meaning) the latter is hydrolysed in an alcaline medium to produce an amino acid of formula I

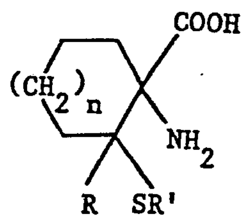

(I)

(in which the substituents R, R′ and n have the above definition) which may be submitted to hydrogenolysis so as to produce a thio derivative of formula VI

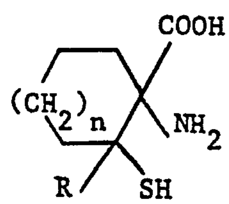

(VI)

17. A process according to claim 16 wherein an amphoteric compound of formula I is converted into an addition salt of a mineral or organic acid or base.

18. A process according to claim 16 wherein the isomers of the compounds of formula I are separated by means of a physical method, such as fractional crystallization or chromatography.

**Patentansprüche**

1. $\alpha$-substituierete cyclische Aminosäuren der allgemeinen Formel I

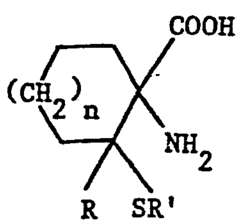

(I)

in der

R ein Wasserstoffatom oder eine niedrigmolekulare Alkylgruppe,

R′ ein Wasserstoffatom, eine niedrigmolekulare Alkylgruppe die möglichenfalls durch eine Hydroxylgruppe substituiert ist, eine monocyclische Arylgruppe, eine Benzylgruppe, eine Phenylgruppe, die durch einem oder mehrere folgender substituente substituiert ist: Halogenatome, niedrigmolekulare Alkoxygruppen, Trifluoromethylgruppen, Aminogruppen, Alkylaminogruppen oder Acylaminogruppen; oder eine Heterocylischegruppe, und

n 0 oder 1 bedeuten.

2. Die Salze der Verbindungen der allgemeinen Formel I nach Anspruch 1 mit vorzugsweise therapeutisch verträglichen anorganischen oder organischen Säuren oder anorganischen oder organischen Basen.

3. Die erythro- und threo-Diastereoisomeren der Verbindungen der allemeinen Formel I nach Anspruch 1 sowie deren optisch aktive Formen.

4. Die 1-Amino-2-benzylthiocyclopentyl-1-carbonsäure.

5. Die 1-Amino-2-mercaptocyclohexyl-1-carbonsäure.

6. Die 2-Methyl-2-benzylthio-1-aminocyclopentyl-1-carbonsäure.

7. Die 1-Amino-2-benzylthiocyclohexyl-1-carbonsäure.

8. Die 1-Amino-2-(p-chlorphenylthio)-cyclohexyl-1-carbonsäure.

9. Die 1-Amino-(thienylthio)- cyclohexyl-1-carbonsäure.

10. Die 1-Amino-2-mercaptocyclopentyl-1-carbonsäure.

11. Die 1-Amino-2-methyl-2-benzylthio-cyclohexyl-1-carbonsäure.

12. Die 1-Amino-2-methylthio-2-methylcyclohexyl-1-carbonsäure.

13. Die 1-Amino-2-(2-hydroxyäthylthio)-2-methylcyclohexyl-1-carbonsäure.

14. Pharmazeutische Zubereitungen, die als Wirkstoff mindestens eine Verbindung nach Anspruch 1 oder eines ihrer Additionssalze mit einer Säure oder einer Base in Mischung mit einem inerten, nichttoxischen, therapeutisch annehmbaren Trägermaterial oder Streckmittel enthalten.

15. Pharmazeutische Zubereitung nach Anspruch 14, dadurch gekennzeichnet, daß ihr Wirkstoffgehalt zwischen 100 mg und 400 mg pro Einzeldosis liegt.

16. Verfahren zur Herstellung der Verbindungen der allgemeinen Formel I nach Anspruch 1

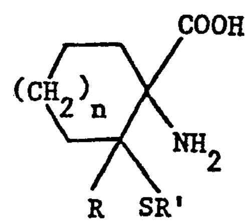

(I)

in der:

R, R' und n die in Anspruch angegebenen Bedentunger besitzen, dadurch gekennzeichnet, daß man ein Cycloalkanon der allgemeinen Formel II

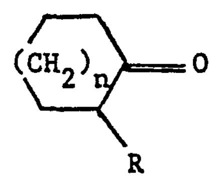

(II)

in der:

R und n die oben angegebenen Bedeutungen besitzen, mit einem Halogenierungsmittel umsetzt, um ein $\alpha$-Halogenketon der allgemeinen Formel III

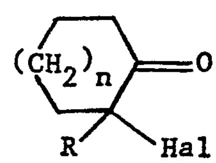

(III)

zu bilden, in der Hal fur ein von Fluor verschiedenes Halogenatom steht und R und n die oben angegebenen Bedeutungen besitzen, das man mit einem Mercaptan der allgemeinen Formel

R' SH

in der:

R' eine niedrigmolekulare Alkylgruppe, eine Arylgruppe oder eine Benzylgruppe darstellt, umsetzt, so daß man das Thioderivat der allgemeinen Formel IV

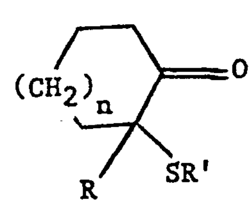

(IV)

erhält, in der die Substituenten R, R' und n die oben angegebenen Bedeutungen besitzen, das man mit einem Alkalmetallcyanid und einem Ammoniumsalz unter den Bedingungen der Strecker-Reaktion umsetzt, so daß man ein Spirohydantoin der allgemeinen Formel V

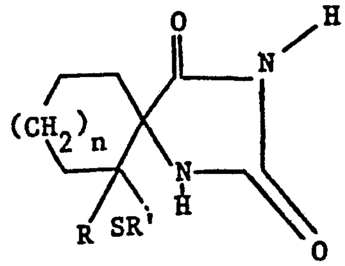

(V)

erhält, in der R, $R_1$ und n die oben angegebenen Bedeutungen besitzen, das man in einem alkalischen Medium Hydrolysiert, so daß man eine Aminosäure der allgemeinen Formel I

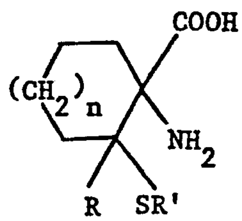

(I)

erhält, in der die Substituenten R, R' und n die oben angegebenen Bedeutungen besitzen, die man gegebenenfalls einer Hydrogenolyse unterwirft, so daß man ein Thioderivat der allgemeinen Formel VI

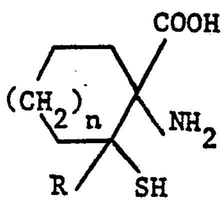

(VI)

erhält.

17. Verfahren nach Anspruch 16, dadurch gekennzeichnet, daß man die Verbindung der allgemeinen Formel I durch Zugabe einer anorganischen oder organischen Säure oder einer anorganischen oder organischen Base in ein Salz unwandelt.

18. Verfahren nach Anspruch 16, dadurch gekennzeichnet, daß man die Diastereoisomeren oder die optischen Isomeren der Verbindungen der allgemeinen Formel I mit Hilfe eines chemischen Reagens' oder einer physikalischen Methode trennt.